# EUROPÄISCHE PATENTSCHRIFT

(11) **EP 3 328 463 B1**
(45) Veröffentlichungstag und Bekanntmachung des Hinweises auf die Patenterteilung: **16.10.2019**
(21) Anmeldenummer: 16744306.8
(22) Anmeldetag: 27.07.2016
(51) Int. Cl.: A61M 1/36

(54) **VERFAHREN ZUR ENTLÜFTUNG EINES DIALYSATORS**
METHOD FOR DEAERATING A DIALYSER
PROCÉDÉ POUR RÉALISER UNE PURGE D'AIR DANS UN DIALYSEUR

(30) Priorität: 29.07.2015 DE 102015009886
(43) Veröffentlichungstag der Anmeldung: 06.06.2018
(73) Patentinhaber: Fresenius Medical Care Deutschland GmbH, 61352 Bad Homburg (DE)
(72) Erfinder: KLÖFFEL, Peter, 97720 Nüdlingen (DE); NÜRNBERGER, Thomas, 97705 Burkardroth (DE)
(74) Vertreter: Herrmann, Uwe
(86) Internationale Anmeldenummer: PCT/EP2016/001301
(87) Internationale Veröffentlichungsnummer: WO 2017/016662

(56) Entgegenhaltungen:
- DE-A1-102011 102 492
- US-A1- 2004 084 371
- US-A1- 2013 150 768

## Beschreibung

Die Erfindung betrifft ein Verfahren zur Entlüftung eines Dialysators und insbesondere zur Entfernung von an der dialysatkammerseitigen Oberfläche der Membran anliegenden Lufteinschlüssen nach Befüllung der Dialysatkammer im Rahmen des Primings. Die Erfindung betrifft ferner ein Dialysegerät mit einer Steuereinheit, auf der ein Algorithmus zur Durchführung eines derartigen Verfahrens hinterlegt ist.

An der Dialysatormembran anliegende Lufteinschlüsse führen dazu, dass an den entsprechenden Stellen während der Dialyse kein Stoffaustausch (Diffusion) stattfinden kann. Es geht somit Membranoberfläche für die Behandlung verloren und die Effektivität (Clearance) der Behandlung reduziert sich.

Vor Durchführung des Dialyseverfahrens erfolgt ein Priming des Dialysators, wobei die Dialysatkammer und die Blutkammer des Dialysators mit einer Primingflüssigkeit befüllt werden. Insbesondere beim Füllen der Dialysatkammer von Kapillardialysatoren tritt oft das Problem von Lufteinschlüssen auf, die an der Dialysatormembran anliegen. Diese Lufteinschlüsse lassen sich nach dem Befüllen nur durch einen Eingriff des Anwenders entfernen. Derartige Primingverfahren sind z.B. aus US 2004/0084371 oder DE 10 2011 102 492 bekannt.

Zur Vermeidung bzw. Entfernung derartiger Lufteinschlüsse ist es bekannt, die Dialysatkammer von unten nach oben befüllen und ggf. zusätzlich am Dialysator zu klopfen. Anschließend ist der Dialysator zu drehen um die Blutkammer zu füllen.

Ein weiteres bekanntes Verfahren sieht ein Befüllen der Dialysatkammer von oben nach unten vor, was den Vorteil hat, dass der Dialysator zum Befüllen der Blutkammer nicht vom Anwender gedreht werden muss. Jedoch ist hier eine vergleichsweise niedrige Befüllgeschwindigkeit wichtig für ein gutes Ergebnis und es verbleibt auch in diesem Fall eine kleine Luftmenge in der Dialysatkammer.

Aufgabe der Erfindung ist es, ein verbessertes Verfahren zur Entlüftung eines Dialysators bereitzustellen.

Diese Aufgabe wird erfindungsgemäß durch ein Verfahren nach Anspruch 1 gelöst. Demnach ist ein Verfahren zur Entlüftung eines Dialysators vorgesehen, der eine Dialysatkammer, eine Blutkammer und eine diese beiden Kammern trennende, semipermeable Dialysatormembran aufweist. Zur Entfernung von an der dialysatkammerseitigen Oberfläche der Membran anliegenden Lufteinschlüssen wird im Rahmen des Verfahrens - nach einer Befüllung der Dialysatkammer und vor einer Befüllung der Blutkammer - in der Dialysatkammer ein Überdruck gegenüber der Blutkammer erzeugt.

Der Überdruck in der Dialysatkammer gegenüber der Blutkammer kann auch dadurch erzeugt werden, dass in der Blutkammer ein Unterdruck erzeugt wird. Er kann beispielsweise auch dadurch erzeugt werden, dass die Blutkammer belüftet ist und in der Dialysatkammer relativ zum Atmosphärendruck Überdruck erzeugt wird.

Unter "Überdruck in der Dialysatkammer" ist zu verstehen, dass der Druck in der Dialysatkammer über dem in der Blutkammer liegt.

Die Befüllung der Dialysatkammer und/oder der Blutkammer erfolgt typischerweise mit einer Primingflüssigkeit, beispielsweise mit einer Dialysierflüssigkeit oder einer Kochsalzlösung.

Da die Blutkammer zum Zeitpunkt der Erzeugung des Überdrucks noch nicht befüllt wurde, ist sie noch gas- und typischerweise luftgefüllt. Daher ist die blutkammerseitige Oberfläche der Membran nicht benetzt. Solange die typischerweise hydrophobe Membran nicht beidseitig benetzt ist, weist sie eine hohe Barrierewirkung für eine wasserbasierte Primingflüssigkeit auf. Daher kann in der Dialysatkammer ein Überdruck aufgebaut werden.

Wenn nun durch unerwünschte Lufteinschlüsse auch Bereiche der dialysatkammerseitigen Oberfläche nicht benetzt sind, ist die Membran vor Erzeugung des Überdrucks bereichsweise - nämlich unterhalb der Lufteinschlüsse - noch trocken. Im derartigen trockenen Zustand weist die typischerweise hydrophobe Membran eine hohe Gas- und insbesondere Luftdurchlässigkeit auf.

Daher können bei herrschendem Überdruck an der dialysatkammerseitigen Oberfläche der Membran anliegende Lufteinschlüsse durch die Membran in die noch nicht befüllte Blutkammer gedrückt und abgeführt werden.

Die Dialysatormembran ist also zusammenfassend - solange sie noch trocken ist und das ist bei Lufteinschlüssen der Fall - für Luft durchlässig. Überdruck in der Dialysatkammer sorgt dafür, dass die Restluft durch die Membran in die noch leere Blutkammer gedrückt wird. Gegenüber einigen bekannten flussgesteuerten Befüllverfahren, die darauf abzielen, Lufteinschlüsse in der Dialysatkammer möglichst gering zu halten, kann durch ein schnelleres Befüllen und die Nachschaltung des erfindungsgemäßen Verfahrens (druckgesteuertes Befüllverfahren) ein Zeitgewinn bei verbesserter Entlüftung erreicht werden.

Bei der semipermeablen Dialysatormembran handelt es sich vorzugsweise um eine Membran, die zumindest im trockenen Zustand hydrophobe Eigenschaften aufweist.

In einer Ausführungsform liegt der Überdruck im Bereich bis 2 bar. Bevorzugt ist ein Überdruck zwischen 50 und 500 mmHg. Bevorzugte Bereiche umfassen den Bereich von zwischen 140 und 220 mmHg und insbesondere den Bereich von zwischen 175 und 195 mmHg. Diese Überdrücke sind zum Drücken der Lufteinschlüsse durch die Membran ausreichend und sind gleichzeitig niedrig genug, um keine Beschädigung am Filter hervorzurufen.

In einer Ausführungsform wird die Blutkammer während der Erzeugung des Überdrucks zur Umgebung hin belüftet. Wenn die Blutkammer zur Atmosphäre hin belüftet wird, kann die übertretende Restluft entweichen und sich somit-kein Gegendruck aufbauen.

In einer Ausführungsform ist die Dialysatkammer während der Durchführung des Verfahrens zu- und/oder rücklaufseitig an ein fluidführendes System angeschlossen. Vorzugsweise ist vorgesehen, dass die Dialysatkammer in den Dialysatkreislauf eines Dialysegerätes integriert ist.

Es kann vorgesehen sein, dass während der Durchführung des Verfahrens auch die Blutkammer bereits in den Blutkreislauf eines Dialysegerätes integriert ist.

Das Verfahren wird vorzugsweise während des Primingvorgangs der fluidführenden Leitungen in einem Dialysegerät durchgeführt. Vorzugsweise wird das Verfahren automatisch oder nach Benutzereingabe anhand einer Steuereinheit des Dialysegerätes ausgeführt.

In einer Ausführungsform wird der Überdruck durch das Schließen eines rücklaufseitig der Dialysatkammer angeordneten Ventils und die zeitgleiche Förderung weiterer Flüssigkeit von der Vorlaufseite des fluidführenden Systems in die Dialysatkammer erzeugt.

Die Förderung weiterer Flüssigkeit von der Vorlaufseite des fluidführenden Systems in die Dialysatkammer kann durch eine Pumpe und/oder durch ein Bilanzierungssystem, wie etwa eine Bilanzkammer erfolgen, die im Dialysegerät ohnehin vorhanden ist. Insbesondere kann eine in der Vorlaufleitung angeordnete Versorgungspumpe verwendet werden. Sofern während des Primings bzw. Spülens ein geschlossener Kreislauf gebildet wird, ist auch die Verwendung einer in der Rücklaufleitung des Dialysators befindlichen Ultrafiltrations- bzw. Rücklaufpumpe denkbar, sofern sie dafür geeignet sind.

Als Rücklaufventil kann in einer Ausführungsform jedes beliebige rücklaufseitig des Dialysators im Dialysatkreislauf angeordnete Ventil dienen. Es kann ein Ventil verwendet werden, dass ohnehin in der Rücklaufleitung vorhanden ist oder ein Ventil, das eigens zur Durchführung des erfindungsgemäßen Verfahrens vorgesehen ist.

Eine Druckmessung in der Dialysatkammer kann beispielsweise durch einen zwischen Pumpe und Dialysatkammer, zwischen Dialysatkammer und Ventil oder am Dialysator selbst angeordneten Drucksensor erfolgen.

In einer Ausführungsform wird der Druckaufbau nach Erreichen eines vorbestimmten Überdruckwerts in der Dialysatkammer gestoppt. Dies kann beispielsweise durch Stoppen der Pumpe erreicht werden. Der vorbestimmte Überdruckwert kann sich in den zuvor beschriebenen Bereichen bewegen.

In einer Ausführungsform wird der Überdruck in der Dialysatkammer nach dem Stoppen des Druckaufbaus über einen Entlüftungszeitraum hinweg gehalten. Dies kann beispielsweise durch Geschlossenhalten des Ventils erreicht werden. Das Halten des Überdrucks bedeutet nicht, dass der Druck in der Dialysatkammer über den Entlüftungszeitraum hinweg konstant bleibt. Vielmehr ergeben sich beim Entweichen von an der Membran anliegenden Lufteinschlüssen oder anderen Einflüssen Druckabfälle.

In einer Ausführungsform werden der in der Dialysatkammer herrschende Druck und/oder dessen Verlauf während des Druckaufbaus gemessen. Alternativ oder zusätzlich kann der in der Dialysatkammer herrschende Druck und/oder dessen Verlauf im Entlüftungszeitraum gemessen werden.

In einer Ausführungsform wird eine Charakteristik des gemessenen Druckverlaufs im Entlüftungszeitraum dazu verwendet, wahlweise eine Wiederholung des Verfahrens oder eine Fortsetzung des Primingvorgangs zu initiieren. Unter der Fortsetzung des Primingvorganges ist beispielsweise ein im Anschluss an das erfindungsgemäße Verfahren erfolgender Spülvorgang der Dialysatkammer oder ein im Anschluss an das erfindungsgemäße Verfahren erfolgendes Befüllen der Blutkammer zu verstehen.

Ferner kann vorgesehen sein, dass die Charakteristik dazu verwendet wird, ein Signal auszugeben, das repräsentativ für den Entlüftungszustand der Dialysatkammer ist.

Bei der Charakteristik kann es sich um die Steigung der Druck-Zeit-Kurve, die Dauer bis zu einem teilweisen oder vollständigen Abbau des Überdrucks oder dergleichen handeln.

Die angegebenen Schritte können von der Steuereinheit des Dialysegerätes automatisch durchgeführt werden.

Alternativ oder zusätzlich kann beispielsweise auch gravimetrisch bestimmt werden, ob in der Dialysatkammer noch Lufteinschlüsse vorhanden sind oder nicht.

In einer Ausführungsform wird eine mit dem gemessenen Druckverlauf während des Druckaufbaus in Verbindung stehende Charakteristik mit einem korrespondierenden Stabilitätskriterium verglichen, das repräsentativ für die Integrität der Dialysatormembran und/oder die Integrität von Schnittstellen der Dialysatkammer mit dem fluidführenden System ist. Bei einer Abweichung der Charakteristik vom Stabilitätskriterium wird vom Gerät ein Warnsignal ausgegeben.

Bei der Charakteristik kann es sich beispielsweise um die maximale Flüssigkeitszufuhr handeln, die zum Aufbau eines bestimmten Drucks in der Dialysatkammer notwendig ist.

Das erfindungsgemäße Verfahren wird jedenfalls vor Beginn einer Dialysebehandlung durchgeführt, wenn der Patient noch nicht am Gerät angeschlossen ist.

Die Erfindung betrifft des Weiteren ein Dialysegerät mit einem Dialysatkreislauf und einem Dialysator, der eine in den Dialysatkreislauf integrierte Dialysatkammer, eine Blutkammer und eine diese beiden Kammern trennende, semipermeable Dialysatormembran aufweist. Das Dialysegerät zeichnet sich erfindungsgemäß dadurch aus, dass es eine Steuereinheit aufweist, die ausgebildet ist, ein erfindungsgemäßes Verfahren durchzuführen.

Weitere Einzelheiten und Vorteile der Erfindung ergeben sich aus dem nachfolgend anhand der Figuren beschriebenen Ausführungsbeispiel. In den Figuren zeigen:
- Figur 1:: eine schematische Darstellung des flüssigkeitsführenden Systems eines Dialysegerätes; und
- Figur 2:: den zeitlichen Verlauf des Flüssigkeitsdrucks in der Dialysatkammer während der Durchführung eines erfindungsgemäßen Verfahrens.

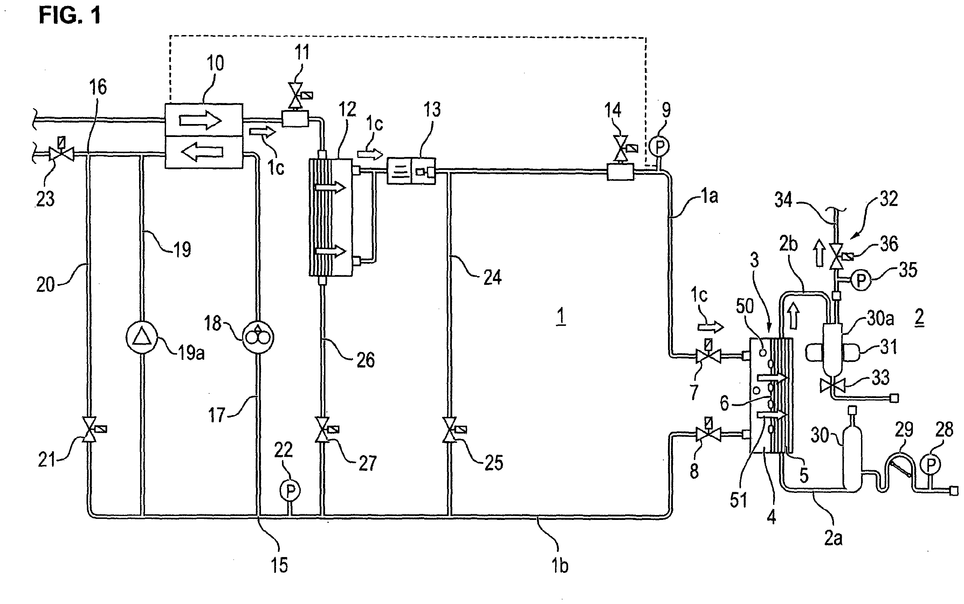

Figur 1 zeigt eine schematische Darstellung des flüssigkeitsführenden Systems eines Dialysegerätes.

Das flüssigkeitsführende System umfasst einen Dialysatkreislauf 1, einen Blutkreislauf 2 und einen Dialysator 3. Der Dialysator umfasst eine Dialysatkammer 4, eine Blutkammer 5 und eine semipermeable Membran 6, welche die Dialysatkammer 4 und die Blutkammer 5 voneinander trennt. In den typischerweise zum Einsatz kommenden Kapillardialysatoren wird die Blutkammer 5 durch die Gesamtheit der Innenvolumina der Hohlfasern gebildet und die Dialysatkammer 4 durch den die Hohlfasern umgebenden Innenraum des Dialysatorgehäuses.

Die Flussrichtung der Priming- bzw. Dialysierflüssigkeit im Dialysatkreislauf ist in der Figur mit den Pfeilen 1c symbolisiert.

In der Vorlaufleitung 1a des Dialysatkreislaufs 1 befindet sich ein Vorlaufventil 7 und in der Rücklaufleitung 1b ein Rücklaufventil 8. Die beiden Ventile sind in der gezeigten Ausführungsform in unmittelbarer Nähe des Dialysators 4 angeordnet und stellen jeweils den nächstkommenden Aktor dar. In der Vorlaufleitung 1a des Dialysators 3 ist ein Vorlaufdrucksensor 9 angeordnet, der zur Bestimmung des vorlaufseitigen Flüssigkeitsdrucks und - im Falle eines geschlossenen Rücklaufventils 8 - des Flüssigkeitsdrucks in der Dialysatkammer 4 des Dialysators 3 dient.

Die Vorlaufleitung 1a umfasst des Weiteren - in der genannten Reihenfolge in Strömungsrichtung - die Vorlaufseite einer Bilanzkammer 10, ein Entlüftungsventil 11, einen Sterilfilter 12, eine Leitfähigkeits- und Temperaturmessanordnung 13 und ein Halteventil 14 zur etwaigen Durchführung eines Druckhaltetests. Der Vorlaufdrucksensor 9 ist zwischen dem Halteventil 14 und dem Vorlaufventil 7 angeordnet.

Die Rücklaufleitung 1b ist zwischen einem ersten Verzweigungspunkt 15 und einem zweiten Verzweigungspunkt 16 dreigeteilt, wobei ein erster Ast 17 - in der genannten Reihenfolge in Strömungsrichtung - eine Rücklaufpumpe 18 und die Rücklaufseite der Bilanzkammer 10 umfasst. Ein zweiter Ast 19 umfasst eine Ultrafiltrationspumpe 19a und führt nicht durch die Bilanzkammer 10. Ein dritter Ast 20 umfasst ein einfaches Hilfsventil 21. Vor dem ersten Verzweigungspunkt 15 ist ein Rücklaufdrucksensor 22 angeordnet. Nach dem zweiten Verzweigungspunkt 16 ist ein Sperrventil 23 angeordnet.

Zwischen der Vorlaufleitung 1a und der Rücklaufleitung 1b ist ferner eine Bypassleitung 24 umfassend ein Bypassventil 25 angeordnet. Die Bypassleitung 24 zweigt zwischen der Messanordnung 13 und dem Halteventil 14 von der Vorlaufleitung 1a ab und mündet zwischen dem Rücklaufventil 8 und dem Rücklaufdrucksensor 22 in die Rücklaufleitung 1b.

Des Weiteren ist eine Retentatleitung 26 mit einem Retentatventil 27 vorgesehen, die die Retentatseite des Sterilfilters 12 mit der Rücklaufleitung 1b verbindet. Die Retentatleitung mündet zwischen der Bypassleitung 24 und dem Rücklaufdrucksensor 22 in die Rücklaufleitung 1b.

Eine in der Figur nicht näher dargestellte, vorlaufseitig der Bilanzkammer 10 in der Vorlaufleitung 1a angeordnete Versorgungspumpe dient dazu, die Priming- bzw. Dialysierflüssigkeit in die Vorlaufleitung 1a und den Dialysator zu fördern und - im Rahmen des erfindungsgemäßen Verfahrens - Druck in der Dialysatkammer aufzubauen.

Der Blutkreislauf 2 umfasst eine arterielle Leitung 2a und eine venöse Leitung 2b, wobei der Dialysator 3 im Gegenstromprinzip geschaltet ist. Die arterielle Leitung 2a umfasst - in der genannten Reihenfolge in Strömungsrichtung - einen arteriellen Drucksensor 28, eine Blutpumpe 29 und ggf. eine arterielle Blasenfalle 30. Die venöse Leitung 2b umfasst - in der genannten Reihenfolge eine venöse Blasenfalle 30a nebst Blasendetektor 31 und Entlüftungsvorrichtung 32 sowie eine venöse Klemme 33. Die Entlüftungsvorrichtung 32 umfasst eine Entlüfungsleitung 34, einen Entlüftungsdrucksensor 35 und ein Entlüftungsventil 36.

Vor Durchführung des erfindungsgemäßen Verfahrens zur Entfernung von Lufteinschlüssen wird die Dialysatkammer 4 des Dialysators 3 im Rahmen eines bekannten Verfahrens gefüllt. Im gezeigten Ausführungsbeispiel erfolgt das Befüllen im Rahmen eines online-Verfahrens, wobei der Dialysator 3 an den Dialysatkreislauf 1 des Dialysegerätes angeschlossen ist. Alternativ kann das Befüllen auch anhand eines Kochsalzbeutels erfolgen.

Das Verfahren beginnt nach dem Befüllen der Dialysatkammer 4 und vor dem Befüllen der Blutkammer 5. Der Blutkreislauf 2 einschließlich der Blutkammer 5 ist bei Durchführung des Verfahrens noch mit Luft gefüllt.

Im Rahmen des Verfahrens wird zunächst das Vorlaufventil 7 offen gehalten und das Rücklaufventil 8 geschlossen. Es wird ein druckgesteuertes Füllprogramm gestartet, das anhand der Versorgungspumpe zusätzliche Primingflüssigkeit durch die Vorlaufleitung 1a in die Dialysatkammer 4 fördert. Da das Rücklaufventil 8 geschlossen ist, baut sich in der Dialysatkammer 4 gegenüber der Blutkammer 5 - in der Umgebungsdruck herrscht - ein Überdruck auf.

Der im Verhältnis zum Umgebungsdruck entstehende Überdruck wird anhand des Vorlaufdrucksensors 9 erfasst. Ist ein definierter Überdruck von beispielsweise 150 mmHg erreicht, wird der Flüssigkeitszulauf gestoppt und während eines Entlüftungszeitraums der am Vorlaufdrucksensor 9 gemessene Druckverlauf bzw. Druckabfall beobachtet. Strömt Luft aus der Dialysatkammer 4 durch die Membran 6 in die Blutkammer 5, so nimmt der Überdruck in der Dialysatkammer 4 schnell wieder ab, da die trockene Membran 6 für Luft einen relativ geringen Flusswiderstand darstellt.

Die Luftblasen sind in der Figur 1 mit dem Bezugszeichen 50 gekennzeichnet und die Strömungsrichtung von der Dialysatkammer 4 in die Blutkammer 5 mit einem Pfeil 51 angedeutet.

Damit sich in der Blutkammer 5 kein Gegendruck zur Atmosphäre aufbaut, ist die Blutkammer 5 zu Atmosphäre hin belüftet, beispielsweise anhand der Enden der arteriellen Leitung 2a und/oder venösen Leitung 2b oder anhand der Entlüftungsleitung 34 bei geöffnetem Entlüftungsventil 36.

Fällt der Überdruck in der Dialysatkammer 4 schnell ab, wird dieser erneut aufgebaut. Fällt der Überdruck in der Dialysatkammer 4 nicht bzw. nur sehr langsam ab, bedeutet dies, dass keine Restluft mehr in der Dialysatkammer 4 vorhanden ist bzw. die zunächst vorhandene Restluft bereits weitestgehend in die noch leere Blutkammer 5 gedrückt wurde.

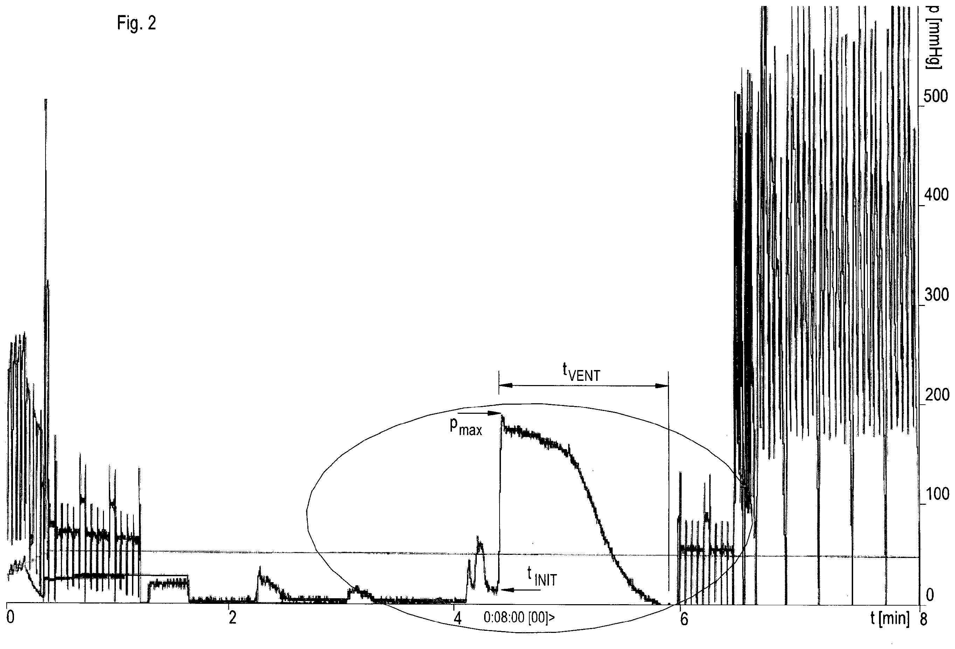

Ein im Rahmen eines Versuchs experimentell bestimmter zeitlichen Verlauf des Flüssigkeitsdrucks in der Dialysatkammer 4 wird in Figur 2 dargestellt. In dem Diagramm ist auf der Ordinate der am Vorlaufdrucksensor 9 gemessene Flüssigkeitsdruck in der Dialysatkammer 4 in mmHg aufgetragen und auf der Abszisse die Zeit in Minuten. Zum Zeitpunkt t_{INIT} wurde das Rücklaufventil 8 geschlossen und die Versorgungspumpe wurde weiterbetrieben, bis sich in der Dialysatkammer ein Überdruck p_{MAX} von 185 mmHg aufgebaut hatte. Dieser Überdruck baute sich aufgrund des Übertritts von Restluft aus der Dialysatkammer 4 in die Blutkammer 5 relativ schnell innerhalb eines Belüftungszeitaums t_{VENT} von etwa 2 bis 3 Minuten wieder vollständig ab.

Dieses Verhalten ist auch durch hydrophobe Eigenschaften der Membran begünstigt. Solange die Blutkammer 5 des Dialysators 3 noch nicht gefüllt und daher trocken ist, weist die Membrane einen großen Flusswiderstand für die wässrige Primingflüssigkeit auf.

Neben einer Entlüftung kann das erfindungsgemäße Verfahren durch zusätzliche Parametrisierung auch verwendet werden, um die Integrität der Dialysatormembran 6 bzw. der Schnittstellen des Dialysators 3 mit der Vorlaufleitung 1a und der Rücklaufleitung 1b zu ermitteln. Hier wird ein Stabilitätskriterium definiert, bei dem es sich beispielsweise um die maximale Flüssigkeitszufuhr handelt, die zum Aufbau eines bestimmten Drucks notwendig ist. Wird dieses Stabilitätskriterium der Volumenbegrenzung nicht eingehalten, können daraus Rückschlüsse auf eine etwaige Ruptur der Membran 6 oder einer undichten Schnittstelle gezogen werden.

## Patentansprüche

1. Verfahren zur Entlüftung eines Dialysators (3), der eine Dialysatkammer (4), eine Blutkammer (5) und eine diese beiden Kammern trennende, semipermeable Dialysatormembran (6) aufweist,
**dadurch gekennzeichnet,**
**dass** zur Entfernung von an der dialysatkammerseitigen Oberfläche der Membran (6) anliegenden Lufteinschlüssen (50) nach einer Befüllung der Dialysatkammer (4) und vor einer Befüllung der Blutkammer (5) in der Dialysatkammer (4) ein Überdruck gegenüber der Blutkammer (5) erzeugt wird.

2. Verfahren nach Anspruch 1, **dadurch gekennzeichnet, dass** der Überdruck im Bereich bis 2 bar, vorzugsweise im Bereich von zwischen 50 und 500 mmHg, vorzugsweise im Bereich von zwischen 140 und 220 mmHg und weiter vorzugsweise im Bereich von zwischen 175 und 195 mmHg liegt.

3. Verfahren nach einem der vorhergehenden Ansprüche, **dadurch gekennzeichnet, dass** die Blutkammer (5) während der Erzeugung des Überdrucks zur Umgebung hin belüftet ist.

4. Verfahren nach einem der vorhergehenden Ansprüche, **dadurch gekennzeichnet, dass** die Dialysatkammer (4) während der Durchführung des Verfahrens zu- und/oder rücklaufseitig an ein fluidführendes System (1) angeschlossen und vorzugsweise in den Dialysatkreislauf (1) eines Dialysegerätes integriert ist, wobei vorzugsweise vorgesehen ist, dass der Überdruck durch das Schließen eines rücklaufseitig der Dialysatkammer (4) angeordneten Ventils (8) und die zeitgleiche Förderung weiterer Flüssigkeit von der Vorlaufseite (1a) des fluidführenden Systems (1) in die Dialysatkammer (4) erzeugt wird.

5. Verfahren nach einem der vorhergehenden Ansprüche, **dadurch gekennzeichnet, dass** der Druckaufbau nach Erreichen eines vorbestimmten Überdruckwerts (p_{MAX}) in der Dialysatkammer (4) gestoppt wird, vorzugsweise durch das Stoppen der Pumpe (29).

6. Verfahren nach einem der vorhergehenden Ansprüche, **dadurch gekennzeichnet, dass** der Überdruck in der Dialysatkammer (4) nach dem Stoppen des Druckaufbaus über einen Entlüftungszeitraum (t_{VENT}) hinweg gehalten wird, vorzugsweise durch Geschlossenhalten des Ventils (8).

7. Verfahren nach einem der vorhergehenden Ansprüche, **dadurch gekennzeichnet, dass** der in der Dialysatkammer (4) herrschende Druck und/oder dessen Verlauf während des Druckaufbaus und/oder im Entlüftungszeitraum (t_{VENT}) gemessen wird.

8. Verfahren nach Anspruch 7, **dadurch gekennzeichnet, dass** eine Charakteristik des gemessenen Druckverlaufs im Entlüftungszeitraum (t_{VENT}) dazu verwendet wird, wahlweise eine Wiederholung des Verfahrens oder eine Fortsetzung des Primingvorgangs, beispielsweise einen Spülvorgang der Dialysatkammer (4) und/oder ein Befüllen der Blutkammer (5) zu initiieren.

9. Verfahren nach Anspruch 7 oder 8, **dadurch gekennzeichnet, dass** eine mit dem gemessenen Druckverlauf während des Druckaufbaus in Verbindung stehende Charakteristik mit einem korrespondierenden Stabilitätskriterium verglichen wird, das repräsentativ für die Integrität der Dialysatormembran (6) und/oder die Integrität von Schnittstellen der Dialysatkammer (4) mit dem fluidführenden System (1) ist, und dass bei einer Abweichung der Charakteristik vom Stabilitätskriterium ein Warnsignal ausgegeben wird.

10. Dialysegerät mit einem Dialysatkreislauf (1) und einem Dialysator (3), der eine in den Dialysatkreislauf (1) integrierte Dialysatkammer (4), eine Blutkammer (5) und eine diese beiden Kammern trennende, semipermeable Dialysatormembran (6) aufweist,
**dadurch gekennzeichnet,**
**dass** das Dialysegerät eine Steuereinheit aufweist, auf der ein Algorithmus zur Durchführung eines Verfahrens nach einem der vorhergehenden Ansprüche hinterlegt ist.

## Claims

1. A method for venting a dialyzer (3) which has a dialyzate chamber (4), a blood chamber (5) and a semipermeable dialyzer membrane (6) separating these two chambers,
**characterized in that**
an overpressure is generated in the dialyzate chamber (4) with respect to the blood chamber (5) for removing air inclusions (50) lying at the surface of the membrane (6) at the dialyzate chamber side after a filling of the dialyzate chamber (4) and before a filling of the blood chamber (5).

2. A method in accordance with claim 1, **characterized in that** the overpressure lies in the range up to 2 bar, preferably in the range between 50 and 500 mmHg, preferably in the range between 140 and 220 mmHg, and further preferably in the range between 175 and 195 mmHg.

3. A method in accordance with one of the preceding claims, **characterized in that** the blood chamber (5) is vented toward the environment during the generation of the overpressure.

4. A method in accordance with one of the preceding claims, **characterized in that** the dialyzate chamber (4) is connected to a fluid-conducting system (1) at the infeed and/or return side during the carrying out of the method and is preferably integrated into the dialyzate circuit (1) of a dialysis machine, wherein provision is preferably made that the overpressure is generated by the closing of a valve (8) arranged at the return side of the dialyzate chamber (4) and the simultaneous conveying of further fluid from the feed side (1a) of the fluid-conducting system (1) into the dialyzate chamber (4).

5. A method in accordance with one of the preceding claims, **characterized in that** the pressure build-up is stopped after reaching a predefined overpressure value (p_{MAX}) in the dialyzate chamber (4), preferably by stopping the pump (29).

6. A method in accordance with one of the preceding claims, **characterized in that** the overpressure in the dialyzate chamber (4) is maintained over a venting time period (t_{VENT}) after the stopping of the pressure build-up, preferably by keeping the valve (8) closed.

7. A method in accordance with one of the preceding claims, **characterized in that** the pressure prevailing in the dialyzate chamber (4) and/or its development during the pressure build-up and/or in the venting time period (t_{VENT}) is measured.

8. A method in accordance with claim 7, **characterized in that** a characteristic of the measured pressure development in the venting time period (t_{VENT}) is used to selectively initiate a repeat of the method or a continuation of the priming procedure, for example a flushing procedure of the dialyzate chamber (4) and/or a filling of the blood chamber (5).

9. A method in accordance with claim 7 or claim 8, **characterized in that** a characteristic related to the measured pressure development during the pressure build-up is compared with a corresponding stability criterion which is representative for the integrity of the dialyzer membrane (6) and/or the integrity of interfaces of the dialyzate chamber (4) with the fluid-conducting system (1); and **in that** a warning signal is emitted on a deviation of the characteristic from the stability criterion.

10. A dialysis machine having a dialyzate circuit (1) and a dialyzer (3) which has a dialyzate chamber (4) integrated into the dialyzate circuit (1), a blood chamber (5) and a semipermeable dialyzer membrane (6) separating these two chambers,
**characterized in that,**
the dialysis machine has a control unit on which an algorithm is stored for carrying out a method in accordance with one of the preceding claims.

## Revendications

1. Procédé pour réaliser une purge d'air dans un dialyseur (3), qui comporte un compartiment à dialysat (4), un compartiment à sang (5) et une membrane de dialyseur (6) semi-perméable séparant ces deux compartiments,
**caractérisé en ce que**,
pour éliminer des inclusions d'air (50) contiguës à la surface côté compartiment à dialysat de la membrane (6), une surpression par rapport au compartiment à sang (5) est générée dans le compartiment à dialysat (4) après un remplissage du compartiment à dialysat (4) et avant un remplissage du compartiment à sang (5).

2. Procédé selon la revendication 1, **caractérisé en ce que** la surpression est comprise dans la plage allant jusqu'à 2 bar, de préférence dans la plage de 50 à 500 mmHg, de préférence dans la plage de 140 à 220 mmHg et plus préférentiellement dans la plage de 175 à 195 mmHg.

3. Procédé selon l'une des revendications précédentes, **caractérisé en ce que**, pendant la génération de la surpression, le compartiment à sang (5) est ouvert à la pression atmosphérique.

4. Procédé selon l'une des revendications précédentes, **caractérisé en ce que**, pendant l'exécution du procédé, le compartiment à dialysat (4) est raccordé côté arrivée et/ou côté retour à un système conducteur de fluide (1) et est de préférence intégré dans le circuit de dialysat (1) d'un appareil de dialyse, la surpression étant de préférence générée par la fermeture d'une vanne (8) disposée côté retour du compartiment à dialysat (4) et le transport simultané de liquide supplémentaire du côté aller (1a) du système conducteur de fluide (1) au compartiment à dialysat (4).

5. Procédé selon l'une des revendications précédentes, **caractérisé en ce que** la montée de la pression est arrêtée, de préférence par l'arrêt de la pompe (29), après qu'une valeur de surpression prédéfinie (p_{MAX}) a été atteinte dans le compartiment à dialysat (4).

6. Procédé selon l'une des revendications précédentes, **caractérisé en ce que**, après l'arrêt de la montée de la pression, la surpression dans le compartiment à dialysat (4) est maintenue, de préférence en maintenant la vanne (8) fermée, pendant une période de purge d'air (t_{VENT}).

7. Procédé selon l'une des revendications précédentes, **caractérisé en ce que** la pression régnant dans le compartiment à dialysat (4) et/ou son évolution pendant la montée de la pression et/ou pendant la période de purge d'air (t_{VENT}) est mesurée.

8. Procédé selon la revendication 7, **caractérisé en ce qu'**une caractéristique de l'évolution de la pression mesurée pendant la période de purge d'air (t_{VENT}) est utilisée pour déclencher au choix une répétition du procédé ou une poursuite du processus d'amorçage, par exemple un processus de rinçage du compartiment à dialysat (4) et/ou un remplissage du compartiment à sang (5).

9. Procédé selon la revendication 7 ou 8, **caractérisé en ce qu'**une caractéristique liée à l'évolution de la pression mesurée pendant la montée de la pression est comparée à un critère de stabilité correspondant, qui est représentatif de l'intégrité de la membrane de dialyseur (6) et/ou de l'intégrité d'interfaces du compartiment à dialysat (4) avec le système conducteur de fluide (1), et **en ce que**, en cas d'écart entre la caractéristique et le critère de stabilité, un signal d'avertissement est émis.

10. Appareil de dialyse comprenant un circuit de dialysat (1) et un dialyseur (3), qui comporte un compartiment à dialysat (4) intégré dans le circuit de dialysat (1), un compartiment à sang (5) et une membrane de dialyseur (6) semi-perméable séparant ces deux compartiments,
**caractérisé en ce que**
l'appareil de dialyse comporte une unité de commande, sur laquelle est enregistré un algorithme pour exécuter le procédé selon l'une des revendications précédentes.
